# EUROPEAN PATENT APPLICATION

(11) **EP 4 799 562 A1**
(43) Date of publication of application: **02.09.2026**
(21) Application number: 25161064.8
(22) Date of filing: 28.02.2025
(51) Int. Cl.: A61B 5/11, A61B 5/00, A61C 19/045

(54) **MEASUREMENT SYSTEM AND METHOD FOR MEASURING TRAJECTORY OF PATIENT'S MANDIBLE RELATIVE TO HEAD**

(71) Applicant: SpinVision Sp. z o.o., 20-218 Lublin (PL)
(72) Inventor: Guzek, Michal, Zagorze (PL); Lulek, Robert, Jawor (PL); Przychodzen, Jakub, Piaseczno (PL); Jamrozy, Milosz, Marianow (PL)
(74) Representative: AOMB Polska Sp. z.o.o.

(57) **Abstract**

The invention discloses a measurement system to be secured on the patient's head, comprising: a band arranged for positioning on the patient's head, an optical measurement pattern secured to the band and arranged for determining of a position of the patient's head and its vertical axis, and to transmit data about the position of the patient's head, means for mechanical binding of the band with established characteristic anatomic points on the patient's head, an electronic level configured for measurement of tilts of the band from an established plumb line, a communications interface for communication with the patient via signals emitted thereby and perceived by the patient's senses, configured for transmitting to the patient information about a change in an angular position of the head based on the information received from the electronic level, at least one camera for recording the image of the patient's head and recording information about a localization of the patient's head from the optical measurement pattern, data transmission means arranged to transmit the data received from the at least one camera and from the electronic level to an analyser module, and the analyser module arranged to receive and process the transmitted data and record the positioning of the characteristic anatomic points of the patient's head and position, orientation or movement of the patient's mandible. The invention also discloses a method for measurement of the patient's mandibular trajectory relative to the head in real time.

## Description

### Field of the Invention

The invention belongs to the field of measurement systems for recording a patient's mandibular trajectory, and more specifically it concerns a measurement system and a method of measuring the patient's mandibular trajectory in relations to the head and its specific anatomic points.

### Prior Art

Document KR100608997B1 discloses a device for measurement of the jaw movement, capable of measuring the rotational center of the patient's jaw the trajectory of its movement. The device for measurement of the jaw movement is a pair of fixed marking units attached correspondingly to the left side and right side of the patient's face and a controlling means which is connected to a camera and which receives image signal from the camera and subsequently displays it.

Document RU2817471C1 discloses a method for tracing of the movement of the mandible and a device for implementation of the method. Recording of the mandible movements is effected via a machine learning method which enables use of the data obtained from fiducial markers installed on the skin of the mandible.

Document DE4411907A1 discloses a method of determining the axes of rotation in joints with the use of markers tracked by a camera and positioned on the movable portion of the joint to enable movement analysis without direct markers mounted.

Systems known in the prior art are based on measurement devices which comprise physical markers mounted on the body, and more specifically on the skin of the mandible, or directly on the teeth, to enable tracking of the jaw movements in three dimensions with the use of cameras, and then processing thus obtained data in order to determine the position or movement of the mandible. The known systems require numerous elements to be mounted on the patient's head. Moreover, for accurate positioning of the head, mirrors or inclinometers are used.

### Summary of the Invention

It is the aim of the invention to provide a measurement system for measuring patient's mandibular trajectory, to eliminate any necessity to use markers on the skin of the mandible or within the oral cavity of the patient. Another aim is to provide such a measurement system to eliminate the necessity for manual positioning of the patient's head to obtain a correct measurement.

The subject of the invention is a measurement system to be secured to a patient's head, comprising:
a band arranged for positioning on the patient's head,
an optical measurement pattern secured to the band and arranged for determining of a position of the patient's head and its vertical axis, and to transmit data about the position of the patient's head,
means for mechanical binding of a position of the band with established characteristic anatomic points on the patient's head,
an electronic level configured for measurement of tilts of the band from an established plumb line,
a communications interface for communication with the patient via signals emitted thereby and perceived by patient's senses, configured for transmitting to the patient information about a change in an angular position of the head based on information received from the electronic level,
at least one camera for recording an image of the patient's head and recording information about a localization of the patient's head from the optical measurement pattern,
data transmission means arranged to transmit data received from the at least one camera and the electronic level to an analyser module,
an analyser module, arranged to receive and process transmitted data and recording of the positions of the characteristic anatomic points of the patient's head and position, orientation or movement of a patient's mandible.

Preferably, the communications interface comprises at least two communication elements positioned oppositely and configured to indicate side of the head.

In one embodiment, the communications interface is a vibration interface.

In one preferred embodiment, the communications interface is an audio interface.

In one embodiment, the communications interface is an optic interface.

The mechanical binding means are stabilizers arranged to stabilize the cover of the head, and preferably they are supra-aural stabilizers secured to the band.

Preferably, the optical measurement pattern is a QR code.

In one embodiment, it comprises at least two cameras wherein the first camera is arranged to record the image of the front of the patient's head and record information from the optical measurement pattern, while the second camera is arranged to record the image of the side portion of the patient's head.

Preferably, the data transmission means is Bluetooth, wireless network, radio network or a wired transmitter.

The analyser module comprises an image processing model arranged to detect the characteristic anatomic points and the mandible based on a vision image of the patient's head in real time.

Preferably, the analyser module comprises an image processing model arranged to detect the patient's mandibular movement trajectory relative to the patient's head in real time.

A second aspect of the invention is a method of measurement of a patient mandibular movement trajectory relative to a head in real time, comprising the steps of:
a) Sending, to a patient's band, of information about a tilt of his/her head, via a communication interface, and about any possible need for correction of a head position,
b) Recording of an image of the patient's head along with an optical measurement pattern, by at least one camera,
c) Transmitting of the data received from an electronic level and from the at least one camera to an analyser module, via data transmission means,
d) Determining of positions of characteristic anatomic points of the patient's head, a position of a mandible relative to these points, a mandibular trajectory, and scaling of measured values of a head tilt and an opening span of the mandible.

Preferably, steps from a) to d) are carried out in a loop in real time.

In one embodiment, it comprises step d1) of displaying of the feedback to the patient by means of a monitoring device.

### Advantageous Effects of the Invention

The presented object of the invention enables convenient operation by the user and requires solely a band to be placed onto the patient's head and positioned in the line of the vertical axis of the body. There is no need for positioning any additional markers on the patient's skin or inside the oral cavity, or use any external elements such as a mirror or inclinometers. The solution is capable of reading out a neutral head positioning directly from the anatomic points and this considerably simplifies the measurement process and enhances the patient's comfort. The measurement system may be used both at home and in a physician consulting room or physiotherapist's surgery.

The object of the invention makes it possible to inform the patient about improper inclination of his/her head with the use of a communication interface. Such communications is effected via signals transmitted by the communications interface to inform the patient about the side to which an excessively large inclination of the head from the established vertical direction occurred. If the patient, despite the feedback from the communications interface being an optical interface and/or from a monitoring device, maintains the improper position of his/her head, this may suggest a possible visual impairment, such as e.g. lack of binocular vision. It is also possible to stabilize the band additionally with the use of additional supra-aural stabilizers and mechanical binding means of the position of the band, based on the characteristic anatomic points of the patient's skull.

### Brief Description of the Drawings

Now the invention will be presented closer in an advantageous embodiment with reference to the enclosed drawing where
Fig. 1- shows an embodiment of a band, in a front view,
Fig. 2 - shows an embodiment of the band, in a side view.

### Detailed Description of Embodiments of the Invention

The subject solution enables measurement of a patient's mandibular trajectory and at the same time determination of characteristic anatomic points of the patient's head. The solution may be applied at home, in a dentist's surgery, in a rehabilitation surgery and in other medical and scientific facilities. The analyser module used in this solution processes information comprising an image of the patient's head and information about his/her head positioning.

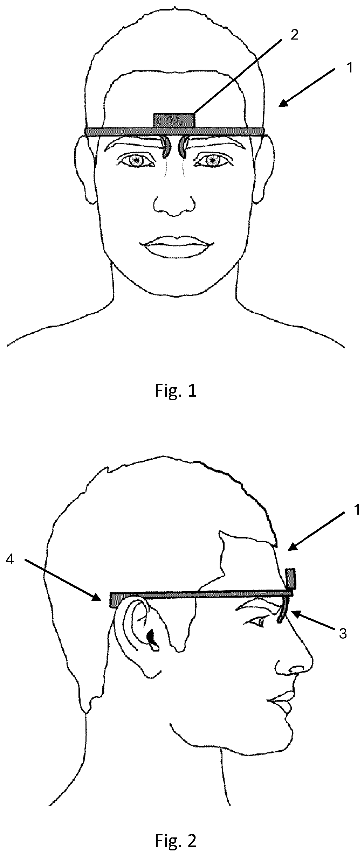

The first aspect of the solution concerns a measurement system to be secured on the patient's head. The measurement system comprises a band 1 and means for mechanical binding of the position of the band 1 with the fixed characteristic anatomic points on the patient's head. The band 1 and the mechanical binding means are shown in Figs. 1 and 2. The band 1 is arranged to be placed on the patient's head, preferably at a circumference of the head. The mechanical binding means are arranged for stationary binding of the band 1 relative to the patient's head.

The wording used in the patent document such as "patient's head" is intended to indicate that the system is linked to specific characteristic anatomical points of the patient which result from the structure of the patient's skull, rather than a generic term for the head. This means that the system is designed to take into account the specific skull anatomy.

Mechanical binding means are configured to enable securing of the band 1 at the characteristic anatomic points of the patient's head, such as ears or nose. The main function of the construction is to ensure secure affixing to the selected points, and this enables accurate use and minimizes the risk of shifting during use.

In one embodiment, the band 1 may be secured on the patient's temporal bone which provides stability thereof and proper positioning. In another option of the construction, the band 1 is provided with a framework 3 to be positioned on the nose and used as a fixed characteristic anatomical point being the root of the nose. The framework 3 to be mounted on the nose root is shown in Figs. 1 and 2.

In another embodiment, as an auxiliary stabilizing point, mastoid process may be also of use which constitutes a fixed characteristic anatomical point. Mastoid process is a good point for securing the band 1 on the head due to its distinct anatomical structure, stability and easy localization. Its hard structure provides solid support.

In another embodiment, mechanical binding means involve simultaneous securing on the temporal bone, nose root and mastoid process.

Additionally, the band 1 may be positioned relative to the axis of the ear canal to enable accurate positioning with regard to the ear structures. In one example, the band 1 is constructed so that a portion 4 of the band is positioned within a natural cavity defined between an auricle and the head, as it may be seen in Fig. 2. In another embodiment, the system comprises stabilizers arranged to secure stably the band 1, preferably, these are supra-aural stabilizers secured on the band which implement positioning of the band 1 relative to the axis of the ear canal. Individual stabilizer is secured with one end to the band 1, and at the other end it has a tip arranged to be placed within a natural cavity present between the auricle and the skull. The band 1 provided with supra-aural stabilizers ensures elimination of movement of the band 1 and stable securing thereof on the patient's head.

In this embodiment, mechanical binding means involve simultaneous securing on the nose root within a natural cavity defined between the auricle and the head.

Preferably, the band 1 is supported on three characteristic anatomical points of the patient's head being the nose root and ear canals.

The measurement system also comprises an optical measurement pattern 2 secured to the band 1, which is arranged to determine the position of the patient's head and its vertical axis and to transmit data about the position of the patient's head. The optical measurement pattern 2 is shown in Figs. 1 and 2. Its positioning, when it is secured on the patient's head, should be on the vertical axis of the patient's head running between the patient's orbits and dividing the head symmetrically into two halves. This positioning ensures the best position for the optical measurement pattern 2 relative to at least one camera which is provided to read out data from the optical measurement pattern 2. The optical measurement pattern 2 should be positioned within a field of view of the camera. The optical measurement pattern 2, upon being read out by at least one camera, provides information about the position of the patient's head.

The optical measurement pattern 2 to be recorded by the camera, provides information about its localization while analysing its position and spatial orientation relative to the field of view of the camera. These data are used to determine specific movement parameters. The optical measurement pattern 2 calibrates the whole arrangement and has a function of a reference arrangement to enable accurate measurement of lengths of the determined segments in the image. This process encompasses precise determining of the distances, dimensions and reciprocal positioning of the elements of the arrangement, such as the camera and the patient. Calibration enables proper reproduction of the real space in the system and this provides high accuracy of records and analysis of the movements of the patient's mandible.

The measurement system also comprises an electronic level device configured for measurements of tilts of the band 1 from an established plumb line. The established plumb line in the electronic level may be described as a reference spatial position which constitutes a reference point for tilt measurement. Angle values recorded by the level are always measured relative to the plumb line. Each tilt from this position is interpreted as an angle change which is monitored and recalculated by the system in real time. Data are recorded in X, Y and Z axes. The electronic level cooperates with a communications interface, and preferably transmits data via Bluetooth. Preferably, the electronic level has a form of a flat batten mounted on the band.

The measurement system also comprises a communications interface for communication with the patient by means of signals emitted thereby and perceived by the patient's senses. As the senses of the patient, in this solution, sight, audition and touch are understood. The communications interface is configured for transmitting to the patient of information about a change in an angular position of the head from the assumed established plumb line position. In the instant of an angular tilt, the communications interface notifies the patient about the irregularity. Preferably, in one example, the intensity of the message is proportional to the angle of the tilt of the head.

In one embodiment, the communications interface includes at least two communications elements positioned opposite to each other, configured to indicate the side of the head. In this example, these at least two communications elements are positioned symmetrically at two sides of the head. One example includes positioning at the front and at the back of the head or at the sides thereof.

Preferably, in another embodiment, in order to indicate precisely the respective tilt, the communications system is uniformly distributed on the entire band. In other examples, it may be distributed in four points: at the sides, at the front and at the back of the band 1.

A preferable example of the communications system is a vibration interface. Vibration interfaces are technologies which use dynamic vibration for specific purposes. Preferably, vibration motors are used in selected points of the band 1. In other embodiments, an audio interface may be used by providing a plurality of speakers in the band, or an optic interface. For an optical interface, the head band 1 may be designed with a framework affixed thereto which protrudes from the front side of the band 1. On the framework, two signal modules are positioned, provided with light sources: one at the left side and the other at the right side. Such configuration provides a better visibility. In another embodiment, the optical interface may be a free standing element with a signalling module provided with sources of light which will illuminate suitably to inform the patient about correction of the head position.

In one embodiment, the communications interface shows an incorrect tilt on the appropriate side. If the head is shifted to the left, the system generates information on the left side of the band 1. If the head is excessively tilted forward, the system generates information at the front of the band 1. If the head is excessively shifted to the right, the system generates information at the right side of the band 1. If the head is excessively tilted rearward, the system generates information at the back of the band 1.

In the embodiment shown, when the communications interface has a form of the vibration interface or the audio interface, it may be distributed on the entire inner surface of the band 1. It is thus possible to identify the four main directions (front, back, left, right), but also intermediate directions, such as left front oblique direction (between front and left sides) and right front oblique direction (between the front and right sides). Analogously, left back and right back oblique directions may be indicated, and this enables providing more accurate information about the head position to be provided.

The electronic level cooperates with the communications interface in real time, and this provides immediate response to tilts. The electronic level, which may comprise sensors, e.g., a gyroscope, accelerometer or another tilt sensor, monitors its position relative to the established plumb line. Upon detection of a tilt exceeding a defined tolerance threshold, it generates a signal to indicate disturbance of the limit. The electronic level transmits the tilt data to a control system positioned within the electronic level, e.g., in a form of an analogue or digital signal. The control system, upon detection that the tilt exceeds a specific value, sends a control signal to the communications interface via a direct electric connection or wireless communication. Upon receipt of the signal the communications interface emits a signal. The length and intensity of the signal may depend on the parameters of the transmitted signal, e.g. a greater tilt = a more intensive signal. The communications interface may provide feedback to the control system to confirm correction of the position or inform on possible errors, e.g., absence of reaction to the signal.

The measurement system also comprises at least one camera for image recording and for recording information about the localization of the patient's head from the optical measurement pattern 2. The camera captures information about the position of the optical measurement pattern 2 by analysing the image in which it is registered. Information about the position of the patient's head is a reference point during measurement of the mandibular movement trajectory.

The measurement system also comprises data transmission means and an analyser module. The data transmission means are arranged to transfer the data obtained from the at least one camera and from the electronic level to the analyser module. The analyser module is arranged to receive and process the transmitted data and to record the position of the characteristic anatomic points of the patient's head as well as position, orientation or movement of the patient's mandible.

The transmission means may be any means to transfer information obtained from the vision module to the analyser module. In various embodiments, the transmission means is Bluetooth, a wireless network, a radio network or a wired transmitter.

In this embodiment, the analyser module comprises an image processing model, suitable to detect characteristic anatomic points and the mandible, based on the vision image of the patient's head in real time as obtained from the at least one camera. The analyser module shows a high precision and reaches an accuracy of up to 0,5 degrees in radians. The analyser module receives input data being an image from the at least one camera and information about the position of the patient's head obtained from the at least one camera which reads out the optical measurement pattern 2. The analyser module also receives input data from the electronic level. In this embodiment, this may be an artificial intelligence model, a mathematical model or a deterministic algorithm, preferably supported by an artificial intelligence model.

The analyser module detects characteristic anatomic points, such as an orbit, each canal, mastoid process, Frankfort plane and the craniovertebral angle (CVA). The module is able to identify with precision these points based on the input data. The orbit is used as one of the key reference points due to its clearly defined anatomic borders. The ear canal has a similar function and provides a reference point in the vicinity of the ear. Such reference planes as the Frankfort plane, assist in determining of the spatial orientation of the entire head, and this is important for correct calibration and operation of the system. The module is able to obtain the Frankfort plane by identification of its constituent points: the lowest point of the bottom edge of the left orbit and the right and left tragions. The module also identifies the position of the mandible.

The craniovertebral angle is used in evaluation of the position of the head and the posture of the cervical segment of the spine. Measurement of CVA involves determining an angle that reflects the position of the head relative to the cervical segment of the spine. It is usually defined as an angle between two lines: the first one runs from the mastoid process of the temporal bone to the mandible, and the other one is a vertical line passing through the seventh cervical vertebra, C7, along the spine.

The analyser module, being the object of the solution, is also able to detect other characteristic anatomical points of the head, not mentioned above.

The analyser module, when detecting characteristic anatomical points, may also define a neutral head position (NHP). The neutral head position is a reference point for evaluation of the position of the head and the cervix, and it has an important role in diagnostics and therapy of muscoskeletal conditions, including headaches, postural disorders and cervical dysfunctions. Neutral head position requires that the axes passing through the ear canals and the lower portions of the orbits are parallel to the horizontal. Reference points such as the point on the nasal root, the centre of the chin and the cervical indentation of the sternum should be on one axis. Additionally, the occipital point in the posteriori part of the skull cannot be shifted forward or backward, and the inner angles of the orbits should define a horizontal line.

Mandibular trajectory is a path along which the mandible travels during its motion. It may be analysed within the context of the respective angles and distances.

In this embodiment, the analyser module comprises a model for image processing, arranged to detect the patient's mandibular trajectory relative to the position of the patient's head in real time. The analyser module is characterized by high precision and reaches an accuracy of up to 0,5 degrees in radians. The analyser module receives input data being: an image from at least one camera and information about the position of the patient's head received from the at least one camera which reads the optical measurement pattern 2. In this embodiment, this may be an artificial intelligence model, a mathematical model or a deterministic algorithm, preferably supported by an artificial intelligence model. The module also identifies the position of the mandible.

In this example, the module detects the mandible movements relative to the patient's head. The movement is detected in the horizontal axis, vertical axis and an axis transverse to the horizontal axis. In the horizontal axis, protrusion and retraction of the mandible are detected. In the axis transverse to the horizontal axis a shift of the mandible to the right or to the left is detected. In the vertical axis descending and ascending of the mandible is detected. Also circular movements are detected. Movement of the mandible is not a linear movement but a complex one, and this means that it encompasses concurrent execution of different kinds of movement within the three-dimensional space. This results from the anatomic and biomechanical structure of the temporomandibular joint. Also values of the mandible opening span are obtained.

Scaling of angles in degrees and obtaining of distances in millimetres within the context of the mandibular movement trajectory involves analysis of the movements of the temporomandibular joints and geometry of the movement thereof. In this case, two main elements should be distinguished: angular changes in the mandible movement and distances resulting from these changes. During the movement of the mandible angles are measured related to the positions of the head. The angles at which the mandible protrudes forward, is retracted or shifted sideways are measured. Scaling of these angles is made in degrees, e.g. with mandible protrusion (extension) when a change in the angle may indicate a change in the mandible position. Also the angle at which the mandible shifts to the right or to the left (lateral movement) is obtained. In this case, this angle may be also measured in degrees and a change in the angle due to the movement may indicate the degree of the mandible shift to the left or to the right. With the obtained positions of the head it is also possible to calculate the distance of the patient's head, and thus optical measurement pattern 2, to the camera. The model also identifies distances in millimetres related to spatial changes in the position of the mandible during movements thereof. The model calculates changes in the distance of the height of the mandible opening span, side movements and forward or backward protrusion of the mandible. A Bennett angle is also measured.

Preferably, the analyser module comprises both models.

Preferably, in this embodiment, the optical measurement pattern 2 is a QR code. At least one camera records video of the QR code in real time. Contrasting areas are detected - black and white squares of the QR code, and three big localization squares which identify a given code and localization thereof. Then information is obtained concerning geometry of the QR code to enable its size and thus orientation. In the analytic module information is included concerning geometry of a given QR code to enable its position and orientation based on the camera readout. The optical measurement pattern 2 in a form of a QR code is contrasted, is characterized by vertical lines mutually perpendicular, a possibility to encode information and presence of right angles in which the localization squares - search patterns are present.

In another embodiment, the vision module comprises at least two cameras, each of which has a field of view positioned along the axes X and Y crossing perpendicularly. The patient wearing the band on his/her head is positioned facing the first camera so that the field of view of the second camera covers the side portion of his head. The first camera is arranged for recording image of the front of the patient's head and recording information from the optical measurement pattern 2, and the second camera is arranged for recording the image of the side portion of the patient's head.

Another aspect of the invention is a method for measuring the patient's mandible relative to the head in real time, comprising the steps of:
a) Sending, to a patient's band 1, of information about a tilt of his/her head, via a communications interface, and about any possible need for correction of the head position,
b) Recording of an image of the patient's head along with an optical measurement pattern 2, by at least one camera,
c) Transmitting of the data received from an electronic level and from the at least one camera to an analyser module, via data transmission means,
d) Determining of positions of characteristic anatomic points of the patient's head, a position of the mandible relative to these points, a mandibular trajectory, and scaling of measured values of the head tilt and an opening span of the mandible.

The method comprises setting a band 1 on the patient's head so that the optical measurement pattern 2 is arranged in the vertical axis of the patient's head. The band 1 is secured via mechanical binding means for the band 1 with established characteristic anatomic points on the patient's head. This ensures stable positioning of the band 1. The method also comprises positioning of the patient in the field of view of a camera to enable reading out data from the optical measurement pattern 2 by at least one camera.

The first step comprises communicating to the patient, and more specifically to the band 1, via a communications interface, of irregularities related to the position of the head, more specifically the tilt thereof. The communications interface also communicates any possible need for correction of the head position. The communication interface receives from the electronic level information about the tilts of the head, the band 1 from the assumed established plumb line.

The second step comprises recording of the patient's head along with the optical measurement pattern 2 by the at least one camera.

The third step comprises transmitting the recorded images from the at least one camera and from the electronic level to an analyser module by data transmission means.

The analyser module, in the fourth step, determines the positions of the characteristic anatomic points of the patient's head, the position of the mandible relative to these points, the mandibular trajectory, and scaling of the measured values of the head tilt and the mandible opening span. Preferably, the analyser module uses an image processing model arranged for detection of the characteristic anatomic points and the mandible based on the vision image of the patient's head in real time and/or an image processing model arranged for detection of the patient's mandibular movement trajectory relative to the patient's head in real time.

Determining of the characteristic anatomic points of the patient's head, preferably, is carried out with the use of models which identify the indication points corresponding to the specific anatomic structures. Based on these points, trajectories and other movement parameters are calculated.

Characteristic anatomic points and positions of the mandible relative to these points are identified in the analyser module and presented in a two-dimensional coordinate system (X, Y). Each point is assigned to a specific position in the plane and this enables accurate analysis of the base and reciprocal positioning of the anatomic structures of the head. Mandibular movement trajectory is recorded in millimetres and angles.

In a preferable embodiment, steps from a) to d) are carried out in a loop in real time. This is aimed at continuous monitoring of the of the patient's mandible operation.

One embodiment comprises step d1) of displaying feedback to the patient via a monitoring device. In this embodiment, the method comprises the use of the monitoring device being an external device. The monitoring device may be a telephone, tablet or laptop computer. It is important for the device to comprise a display large enough to enable the patient to see easily what is happening on the display. Preferably, the analyser module is located in a calculation cloud.

The method described in this solution is to be used during therapy of the patient, both at home and in a physiotherapist's surgery or physician's consulting room. The patient is given a special exercise programme compatible with the monitoring device. The method according to the invention is then performed. After starting the exercise programme on the monitoring device, the patient puts on the band 1 and assumes a sitting position on a chair or a standing position in front of the monitoring device.

It is important that the monitoring device is to be provided with at least one camera executing a function of patient movement tracking system. The camera in the monitoring device is at least one camera as described in the subject solution. Alternatively, the patient may use another camera. During the prescribed exercises with his/her head or mandible the patient receives feedback displayed on the screen to indicate whether the exercises are carried out correctly. Preferably, the position of the established plumb line in the electronic level is automatically established for each exercise in real time. Additionally, the communication interface informs the patient in real time whether the position of the head in a given exercise is correct. This allows the therapy to be more precise and effective.

The feedback may also include a message informing on impossibility to record localization of the optical measurement pattern 2 due to a turn of the patient's head in a manner which causes that the optical measurement pattern 2 is out of the field of vision of the camera.

## Claims

1. A measurement system to be secured to a patient's head, comprising:
a band (1) arranged for positioning on the patient's head,
an optical measurement pattern (2) secured to the band (1) and arranged for determining of a position of the patient's head and its vertical axis, and to transmit data about the position of the patient's head,
means for mechanical binding of a position of the band (1) with established characteristic anatomic points on the patient's head,
an electronic level configured for measurement of tilts of the band (1) from an established plumb line,
a communications interface for communication with the patient via signals emitted thereby and perceived by patient's senses, configured for transmitting to the patient information about a change in an angular position of the head based on information received from the electronic level,
at least one camera for recording an image of the patient's head and recording information about a localization of the patient's head from the optical measurement pattern (2),
data transmission means arranged to transmit data received from the at least one camera and the electronic level to an analyser module,
the analyser module, arranged to receive and process transmitted data and recording of the positions of the characteristic anatomic points of the patient's head and position, orientation or movement of a patient's mandible.

2. The measurement system according to claim 1 **characterized in that** the communications interface comprises at least two communication elements positioned oppositely and configured to indicate side of the head.

3. The measurement system according to claims 1 or 2 **characterized in that** the communications interface is a vibration interface.

4. The measurement system according to claims 1 or 2 **characterized in that** the communications interface is an audio interface.

5. The measurement system according to claims 1 or 2 **characterized in that** the communications interface is an optic interface.

6. The measurement system according to any one of claims from 1 to 5 **characterized in that** the mechanical binding means are stabilizers arranged to stabilize the cover of the head, and preferably they are supra-aural stabilizers secured to the band.

7. The measurement system according to any one of claims from 1 to 6 **characterized in that** the optical measurement pattern (2) is a QR code.

8. The measurement system according to any one of claims from 1 to 7 **characterized in that** it comprises at least two cameras wherein the first camera is arranged to record the image of the front of the patient's head and record information from the optical measurement pattern (2), while the second camera is arranged to record the image of the side portion of the patient's head.

9. The measurement system according to any one of claims from 1 to 8 **characterized in that** the data transmission means is Bluetooth, wireless network, radio network or a wired transmitter.

10. The measurement system according to any one of claims from 1 to 9 **characterized in that** the analyser module comprises an image processing model arranged to detect the characteristic anatomic points and the mandible based on a vision image of the patient's head in real time.

11. The measurement system according to any one of claims from 1 to 10 **characterized in that** the analyser module comprises an image processing model arranged to detect the patient's mandibular movement trajectory relative to the patient's head in real time.

12. A method of measurement of a patient mandibular movement trajectory relative to a head in real time, comprising the steps of:
a) Sending, to a patient's band (1), of information about a tilt of his/her head, via a communication interface, and about any possible need for correction of a head position,
b) Recording of an image of the patient's head along with an optical measurement pattern (2), by at least one camera,
c) Transmitting of the data received from an electronic level and from the at least one camera to an analyser module, via data transmission means,
d) Determining of positions of characteristic anatomic points of the patient's head, a position of a mandible relative to these points, a mandibular trajectory, and scaling of measured values of a head tilt and an opening span of the mandible.

13. The method of measurement according to claim 12, **characterized in that** steps from a) to d) are carried out in a loop in real time.

14. The method of measurement according to claims 12 or 13, **characterized in that** it comprises step d1) of displaying of the feedback to the patient by means of a monitoring device.
